Europäisches Patentamt
European Patent Office
Office européen des brevets

(19) (11) Publication number: **0 309 870 A2**

(12) **EUROPEAN PATENT APPLICATION**

(21) Application number: **88115414.0**

(22) Date of filing: **20.09.88**

(51) Int. Cl.4: **C07C 161/04 , C09K 19/32 , C09K 19/34**

(30) Priority: **25.09.87 PL 267922**

(43) Date of publication of application: **05.04.89 Bulletin 89/14**

(84) Designated Contracting States: **CH DE GB LI**

(71) Applicant: **Wojskowa Akademia Techniczna**
**PL-01-489 Warszawa-49(PL)**

(72) Inventor: **Dabrowski, Roman**
**ul. Buska 40**
**P-02-924 Warszawa(PL)**
Inventor: **Dziaduszek, Jerzy**
**ul. Mlynarska 30a m. 26**
**P-01-171 Warszawa(PL)**
Inventor: **Drzewinski, Witold**
**ul. Tomcia Palucha 6 m. 12**
**P-02-495 Warszawa(PL)**
Inventor: **Szczucinski, Tomasz**
**ul. Rozlogi 9 m. 4**
**P-01-310 Warszawa(PL)**

(74) Representative: **von Füner, Alexander, Dr. et al**
**Patentanwälte v. Füner, Ebbinghaus, Finck**
**Mariahilfplatz 2 & 3**
**D-8000 München 90(DE)**

(54) **Liquid crystalline compounds containing the bicyclo[2,2,2]-octane ring and the phenylisothiocyanate group, their manufacturing and liquid crystalline mixtures containing same.**

(57) New liquid crystalline compounds containing the bicyclo[2,2,2]octane ring and the phenylisothiocyanato group in their molecules of the general formula 1:

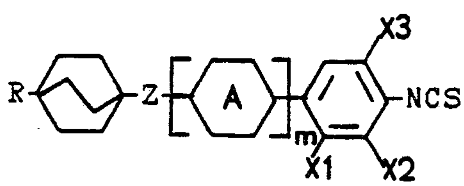

1

wherein: R is a normal or branched alkyl $H_{2n+1}C_n$- or alkenyl $H_{2n-1}C_n$- group containing up to 15 carbon atoms, some of the hydrogen atoms in the alkyl chain being sometimes substituted by a fluorine atom, Z is a single bond or -COO-, -OOC-, $-CH_2O-$ or -C≡C- group, the ring A is a 1,4-disubstituted benzene ring or 2,5-disubstituted pyrimidine ring or a 2,5-disubstituted pyridine ring or a 2,5-disubstituted 1,3-dioxane ring, m stands an integer 0 or 1, X1, X2 and X3 are identical or different and each of them denotes a hydrogen atom or a fluorine atom or a chlorine atom or a bromine atom or a cyano group, however, if m = 1 and the

EP 0 309 870 A2

ring A is not a benzene ring then Z is also a $-CH_2CO-$, $-CO-CH_2-$, $-CH=CH-$, $CHYCH_2-$, $-CH_2-CHY-$, $-C_6H_4-CH_2-CHY-$, $-C_6H_4-CHY-CH_2-$, or $C_6H_4-C\equiv C-$ group where Y is a hydrogen atom or a fluorine atom or a chlorine atom or a cyano group or a methoxy group or a hydroxy group are prepared and used as component of liquid crystal mixture. Method manufacturing of compounds 1, the liquid crystal mixture containing compounds 1 and their use for electrooptical devices are described.

# Liquid crystalline compounds containing the bicyclo[2,2,2]-octane ring and the phenylisothiocyanate group, their manufacturing and liquid crystalline mixtures containing same

The present invention concerns liquid crystalline compounds containing in their molecule the bicyclo-[2,2,2]-octane ring and the phenylisothiocyanato group, and also the methods of manufacturing these compounds, and those of preparing liquid crystalline mixtures containing same applicable in electrooptical indicating elements.

Bi- and tri-nuclear liquid crystalline compounds are known that have in the nucleus of their molecules the bicyclo[2,2,2]octane ring, see United Kingdom Patents 2.027.027, 2.027.708, 2.065.104. As compared with other liquid crystalline compounds without bridging groups those with one or more bicyclooctane rings in the molecule reveal a higher clearing points than the analogous compounds with the cyclohexane, benzene or pyrimidine ring in the molecule, but at the same time they show much higher melting points what makes that their solubility in liquid crystalline mixtures is moderate. The bicyclo[2,2,2]octane derivatives with non-polar substituens in the terminal position, e.g. alkyl groups, are usually smectics, while those with polar substitutents like -CN are nematics. The bicyclo[2,2,2]-octane derivatives with the -CN terminal group show high viscosity, several times higher than their analogs with the cyclohexane or benzene ring. For instance, the ternary mixture of:
1-n-propyl-4-(4 -cyanophenyl)bicyclo[2,2,2]octane,
1-n-pentyl-4-(4 -cyanophenyl-bicyclo[2,2,2]octane, and
1-n-heptyl-4-(4 -cyanophenyl)bicyclo[2,2,2]octane
reveals at 20° C bulk viscosity of $\eta 20^\circ$ = 96 mPa.s, while the viscosity of the analogous mixture composed of three 1-n-alkyl-4-(4′-cyanophenyl)cyclohexanes is $\eta 20^\circ$ = 26 mPa.s, and that of the mixture composed of three 4-n-alkyl-4′-cyanobiphenyls is $\eta 20^\circ$ = 34 mPa.s (M.J. Bradshaw et al., Mol. Cryst. Liq. Cryst., 97, 177 (1983).

Unexpectedly it was found that the substitution of the polar cyano group (-CN) by the isothiocyanato group (-NCS) in bicyclooctane derivatives results in a 3-5 time lowering of viscosity, and in many cases also of melting point ($T_m$) and melting enthalpy, the high clearing point and nematic properties remaining unaffected. The object of the present invention are novel liquid crystalline compounds containing in their molecule the bicyclo[2,2,2]octane ring and the phenylisothiocyanato group described by the general formula:

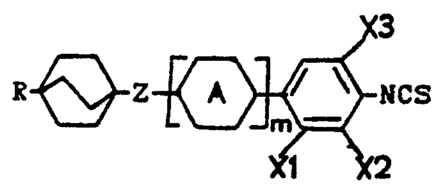

1

where: R is a normal or branched alkyl $H_{2n+1}C_n$- or alkenyl $H_{2n-1}C_n$- group containing up to 15 carbon atoms (some hydrogen atoms in the alkyl chain may be substituted with a fluorine atom), Z is a single bond or -COO- or -OOC-, $-CH_2O-$ or -C≡C- group, A is a 1,4-disubstituted benzene ring or trans-1,4-disubstituted cyclohexane ring or 2,5-disubstituted pyridine ring or 2,5-disubstituted 1,3-dioxane ring, m is an integer 0 or 1, X1, X2 and X3 are identical or different and each of them may be a hydrogen or fluorine or chlorine or bromine atom or cyano group; for m = 1 when A is not a benzene ring, Z is also the $-CH_2CO-$ or $-CO-CH_2-$ or -CH = CH- or $-CHYCH_2-$ or $-CH_2-CHY-$ or $-C_6H_4-CH_2-CHY-$ or $-C_6H_4-CHY-CH_2-$ or $-C_6H_4-C≡C-$ group where Y is hydrogen or fluorine or chlorine atom or cyano or methoxy or hydroxy group.

According to the given definition of the general formula, for m = 0 the compounds with formulae 1a, 1b, 1c, 1d and 1e reveal optimal applicational properties:

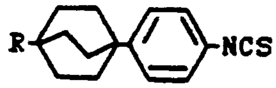
1a

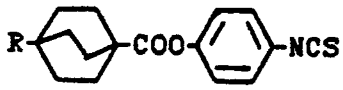
1b

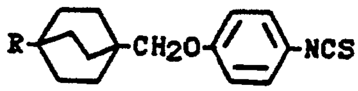
1c

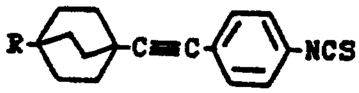
1d

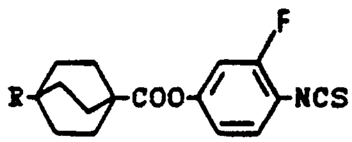
1e

and for m = 1 the optimal properties are revealed by the compounds with formulae 1f, 1g, 1h, 1i, 1j, 1k and 1l:

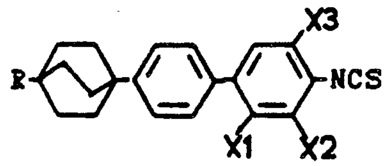 1f

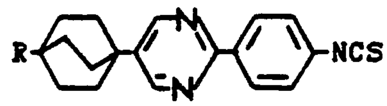 1g

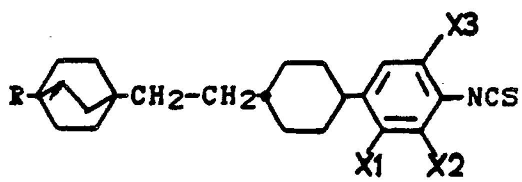 1h

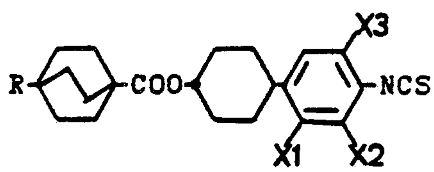 1i

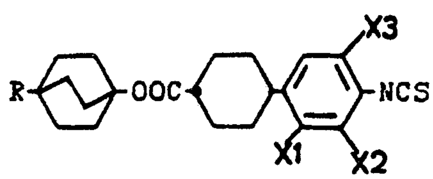 1j

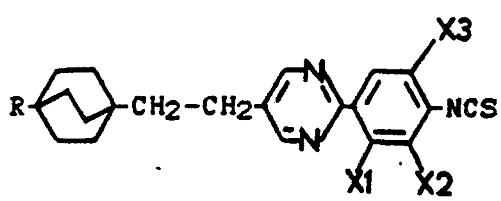 1k

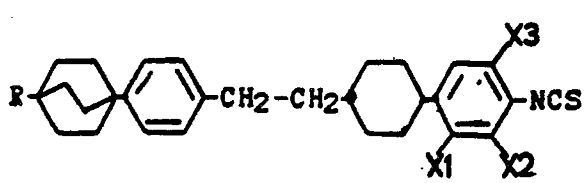 1l

These compounds are favourable components of liquid crystalline compositions used as indicator elements.

The invention also concerns liquid crystalline mixtures including compounds of the general formula 1. The mixtures that are the object of this invention are at least binary mixtures containing at least one compound of the general formula 1. The remaining components of the mixture may be other known liquid

crystalline compounds, optically active compounds, solvents and dyes.

Mixtures with a wide range of the nematic phase can be obtained by mixing binuclear compounds, for instance, the members of the homologous series 1a and/or 1b and/or 1c and/or 1d and/or 1e or together 1a and/or 1b and/or 1c and/or 1d and/or 1e with one or several trinuclear compounds of formula 1 among which 1f, 1g, 1h, 1i, 1j, 1k and 1l are preferred. Compounds of general formula 1 can also be mixed with other liquid crystalline compounds known from the art. Compounds of the general formula 1 when added to mixtures composed of compounds with low clearing points raise effectively the clearing points of these mixtures. Especially favourable mixtures are obtained when compounds of formula 1a, 1b, or 1c are used together with 4-(trans-4 -n-alkylcyclohexyl)-1-isothiocyanatobenzenes of formula 2 (described by us in U.S. Patent 4.528.116) in which they dissolve readily.

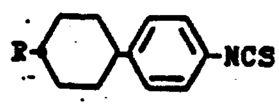

2

The resultant mixtures have a low viscosity at room temperature. In Fig. 1 the variation of viscosity and clearing point are presented of the mixture composed of three 4-(trans-4 -n-alkylcyclohexyl)-1-isothiocyanatobenzenes:
4-(trans-4-propylcyclohexyl)-1-isothiocyanatobenzene 40 wt%
4-(trans-4-hexylcyclohexyl)-1-izothiocyanatobenzene 42 wt%
4-(trans-4-octylcyclohexyl)-1-izothiocyanatobenzene 18 wt%
with the introduced quantity of compound 1a in the form of 4-(4 -n-hexylbicyclo[2,2,2]octyl-1)-isothiocyanatobenzene.
In this way when the clearing point of the base mixture is raised by 10°C its viscosity at 20°C is raised only by 2,2 mPa.s. Mixtures of low viscosity and a very wide range of the mesophase are obtained from compounds of the formula 1a and/or 1b and/or 1c and/or 1d and/or 1e, and of compounds 2 i 3.

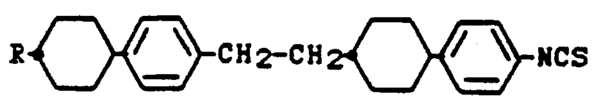

3

The mixtures composed of the three described above compounds are characterized by a narrower interval of clearing temperatures, as distinguished from the mixtures composed of compounds 2 and 3 only. Further favourable components of the mixtures are compounds:

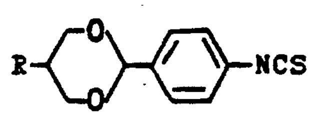

4

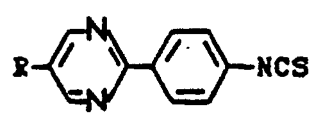

5

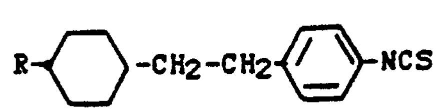

6

as well as compounds that are not isothiocyanates and especially those of the formulae:

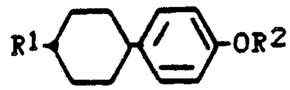

7

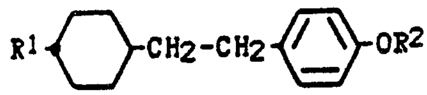

8

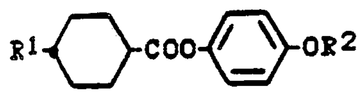

9

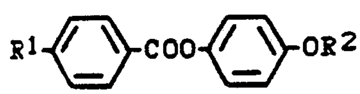

10

in which $R^1$ and $R^2$ are the same or different and denote alkyl chains of 2 to 8 carbon atoms. When mixing compounds 1 with certain non-polar compounds one should expect the induction of the smectic phase to occur. This can be taken advantage of in the preparation of smectic mixtures from nematic compounds , as well as in preparation of mixtures with the smectic and nematic phases of a required temperature range of the nematic phase above the smectic phase. Such properties are revealed by mixtures obtained from compounds 1a-1d and compound 10.

The preferable procedure of obtaining the compounds that are the subject of the present invention consist in treating the amine of formula 11:

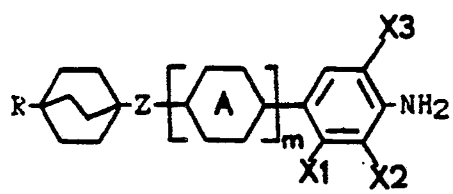

11

where R, Z, A and m have the same meaning as in the general formula 1, with thiophosgene in the presence of a hydrochloride acceptor, i.e. organic or inorganic base or basic salt - preferably tertiary amine, e.g. triethylamine, or calcium or sodium carbonate or calcium hydroxide. A two-step method is also recommended in which amine 11 is treated in an organic solvent, preferably benzene, with carbon disulfide in the presence of a tertiary amine, preferably triethylamine, and the crystalline trialkylammonium dithiocarbamate obtained in the reaction is separated from the reaction mixture by filtration and subsequently treated with ethyl chloroformate in the presence of a tertiary amine. In this reaction the dithiocarbamate is converted to the isothiocyanate of formula 1. The product obtained in the one-step or two-step process is isolated and purified by conventional methods, preferably by crystallization from organic solvents, and in the case of compound 1a or 1c also by distillation under vacuum.

The scheme given below illustrates the described above process obtaining compound 1:

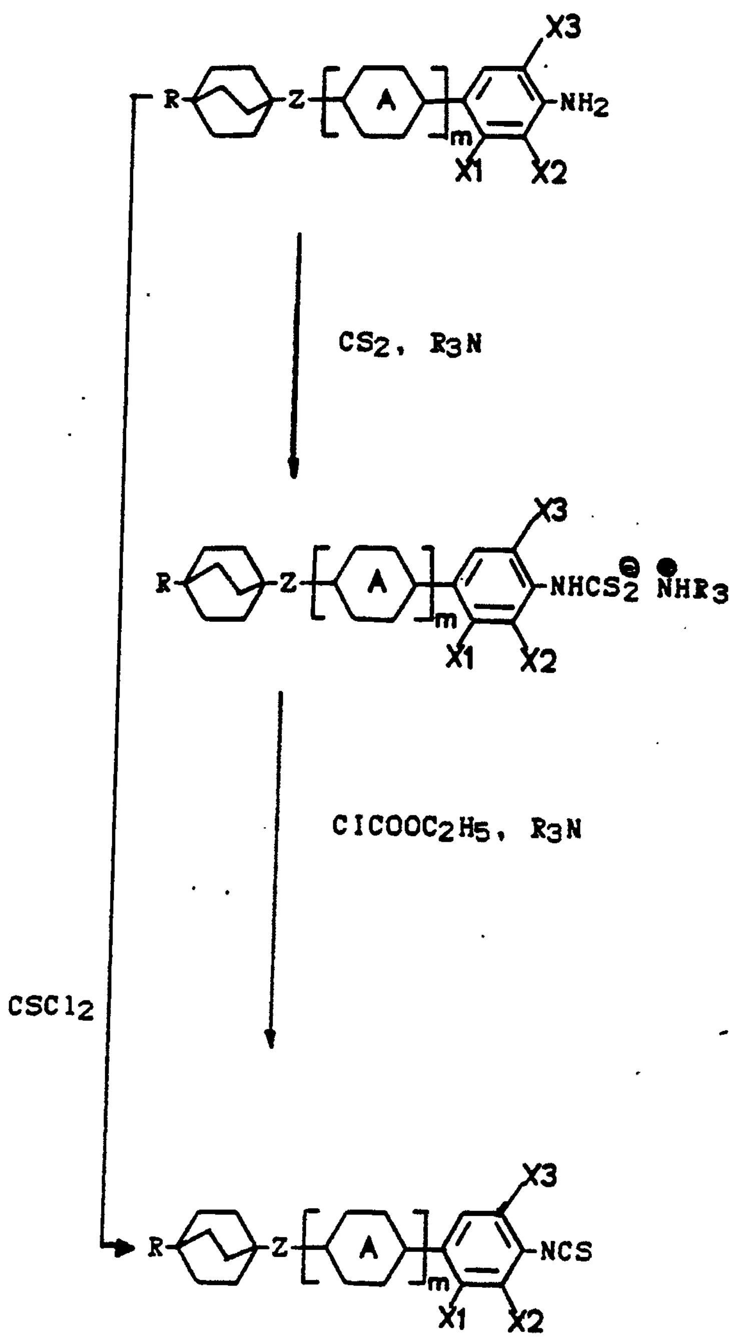


11

12

1

If Z is a single bond, m=0 or m=1, and A is a benzene ring, amine 11 is obtained according to the following scheme:

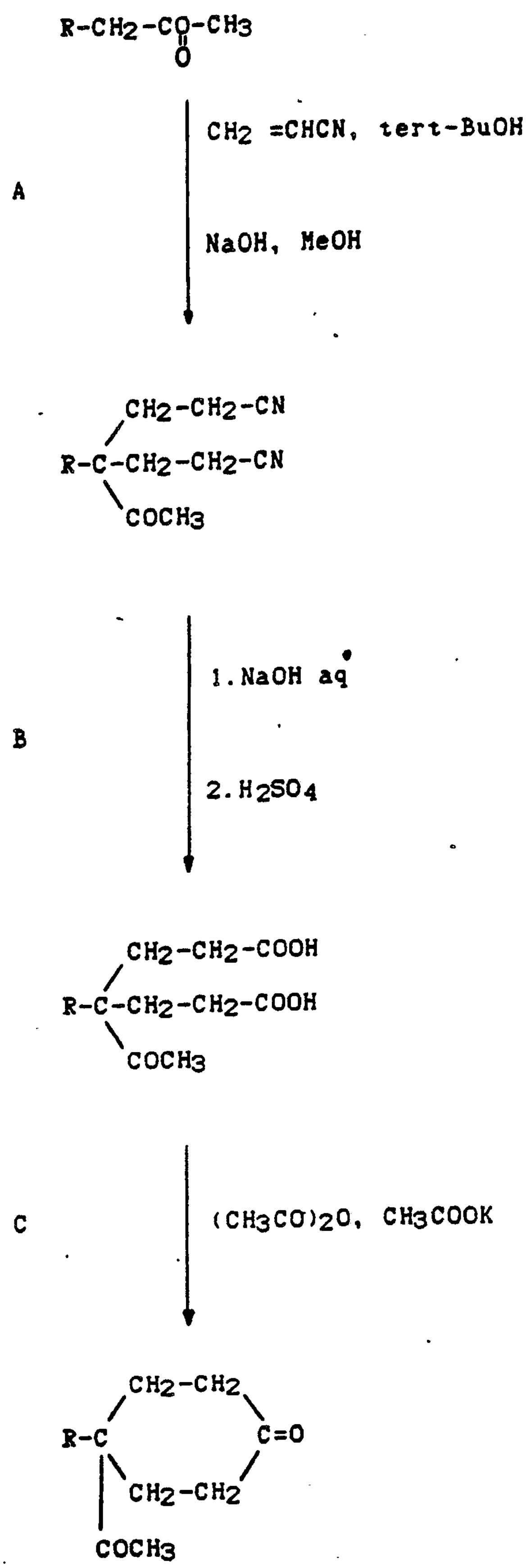
A
CH2 =CHCN, tert-BuOH
NaOH, MeOH
B
1. NaOH aq
2. H2SO4
C
(CH3CO)2O, CH3COOK

13

14

15

16

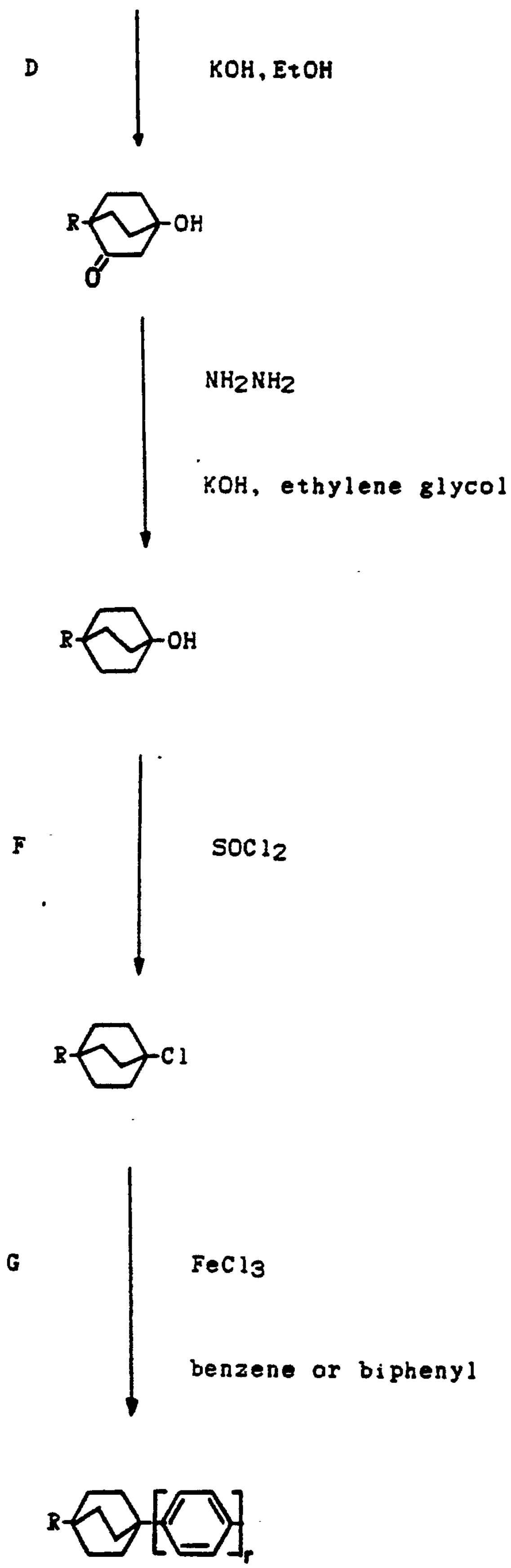
D
KOH, EtOH
NH2NH2
KOH, ethylene glycol
F
SOCl2
G
FeCl3
benzene or biphenyl

17

18

19

20

benzene (r=1), biphenyl (r=2)

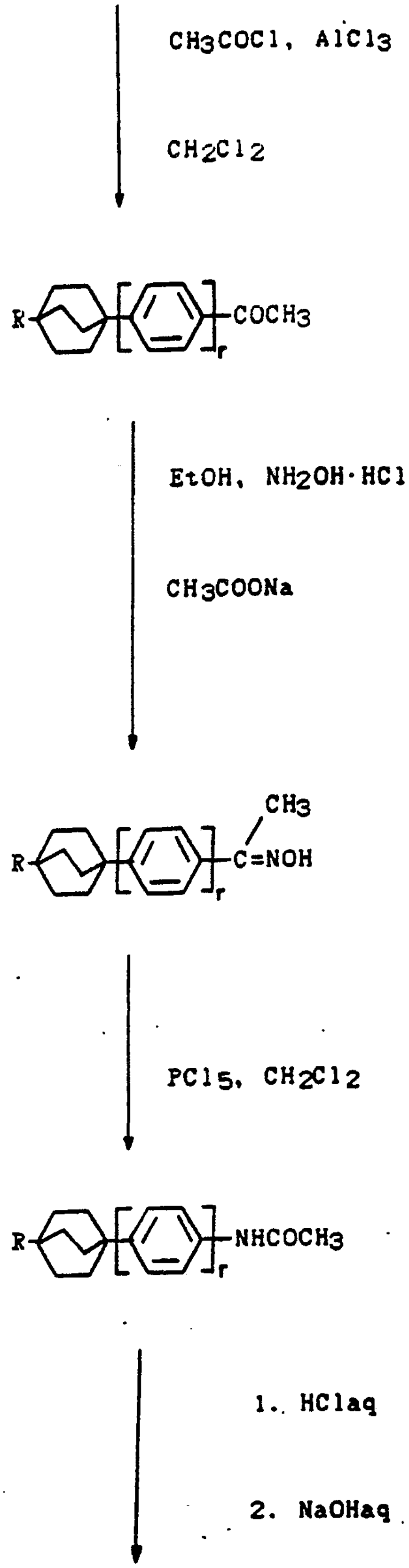


H

21

I

22

J

23

K

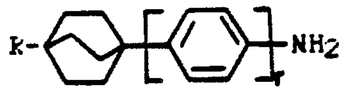

11a, f

The procedure shown in the scheme makes use of generally known methods, however, good results are obtained of the reactions in steps A, B, C, D and E if the method described by Gray'a w J.C.S.Perkin II, 1987 s.26 is used and in step F if the procedure described by P.V.Adomenas in Zh. Org. Khim. 19, 2366 (1983) is applied. To convert 4-alkylbi-cyclo[2,2,2]octyl chloride obtained in step F into 4-alkylbicyclo[2,2,2]-octylbenzene or 4-alkylbicyclo[2,2,2]-octylbiphenyl it is most favourable to react it with benzene or biphenyl in the presence of anhydrous ferrous chloride. Next the hydrocarbons, formula 20 are converted into ketone 21 by reacting with acetyl chloride in the presence of aluminium chloride; next ketone 21 in reaction with hydroxylamine in an acetate buffer solution yields oxime 22 which converts under the action of phosphorus pentachloride in a solvent, favourably dichloromethane, into the respective acetanilide 23 which heated with dilute hydrochloride acid hydrolyses yielding the amine 11a or 11f hydrochloride. If Z a carboxylic group -COO-, amine 11 is preferably obtained according to the following scheme:

24

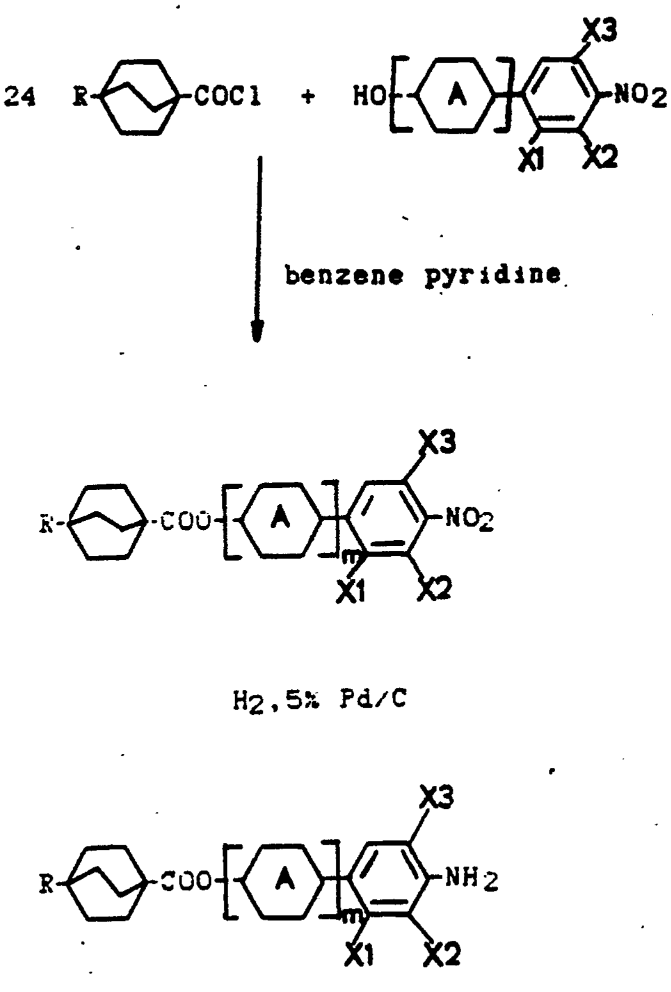


25

26

11

The procedure shown in the scheme consist in treating with 4-n-alkylbicyclo[2,2,2]octane-1-carboxylic acid chloride the respective nitrophenol or nitrophenyl alcohol 25, e.g. p-nitro phenol, in an organic solvent (preferably benzene) with the 4-n-alkylbicyclo[2,2,2]octane-1-carboxylic acid chloride (obtained preferably by treating 4-n-alkylbicyclo[2,2,2]octane-1-carboxylic acid with oxalyl chloride) in the presence of a base, e.g. ternary amine, preferably pyridine. The obtained nitro ester 26 may be converted with a high yield into the respective amino ester 11 if the hydrogen reduction is conducted on a palladium catalyst (5% Pd on carbon) in the conventional way. The relevant acid may easily be obtained by the method described by P.V.

Adomenas, Zh. Org. Khim., 19, 2366 (1983).

The amine of formula 11, if Z is $-CH_2O-$, is obtained according to the scheme below:

27 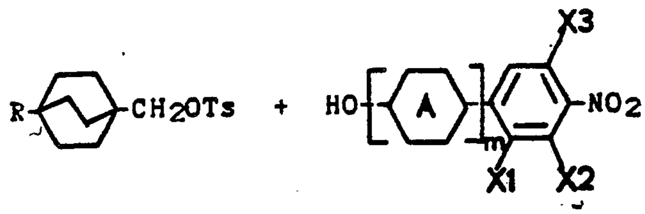 28

29 11
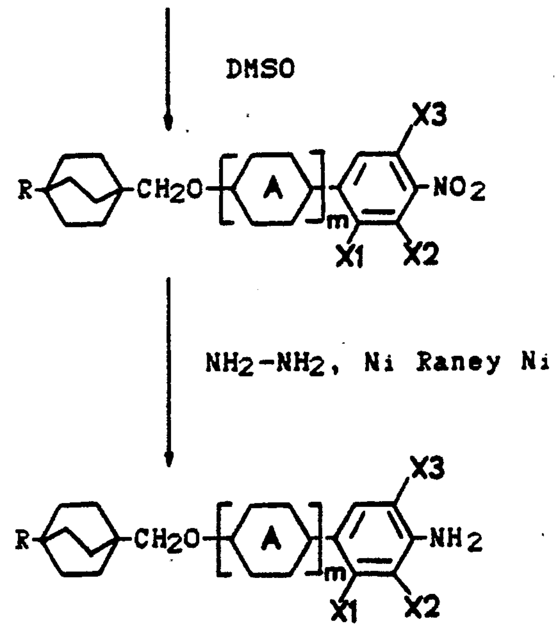


The amine of formula 11, if Z is the $-COCH_2-$, $-CHYCH_2-$ or $-C{\equiv}C-$ group, is obtained according to the scheme:

24 31 32
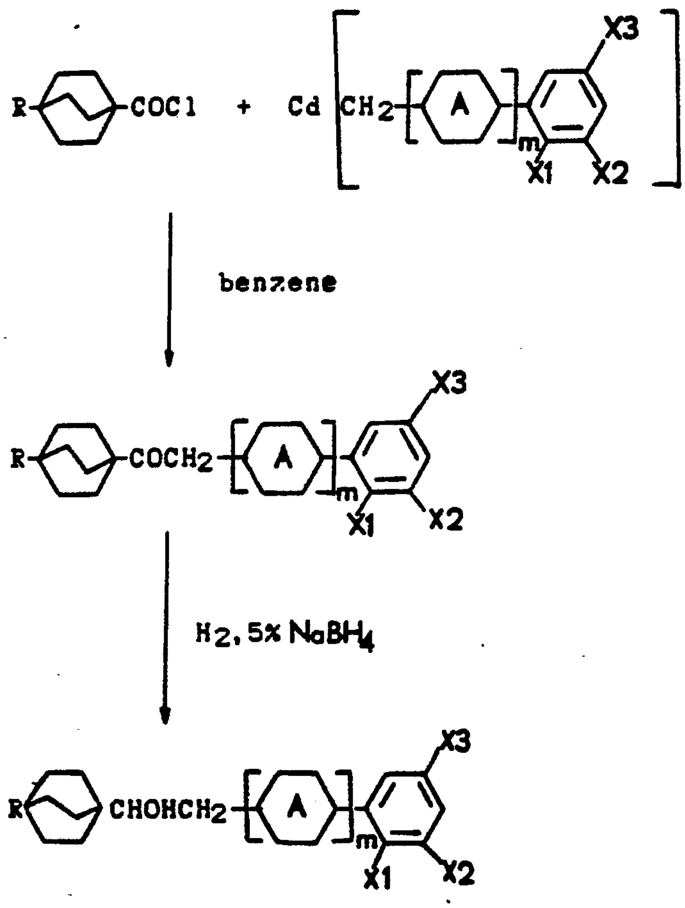

33, 34, 11

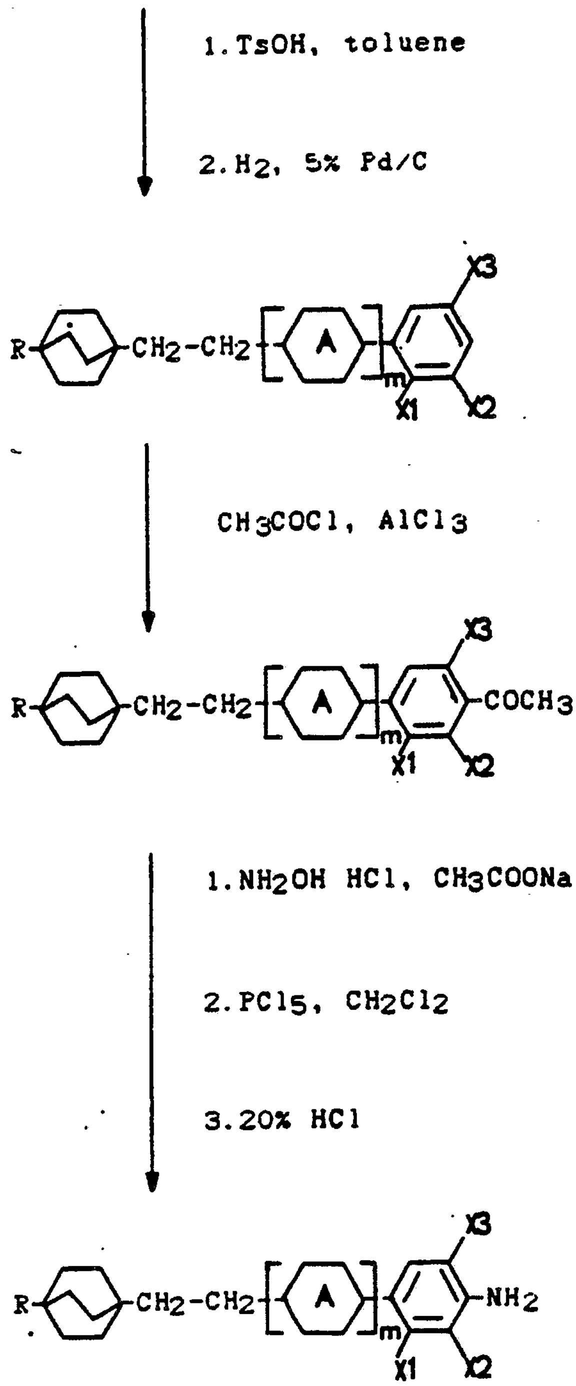


Compound 31 may easily be converted into the derivative with Z= $-CCl_2-CH_2-$ and next into the compound with Z= $-C{\equiv}C-$Compound 32 is a substrate not only for the compound with Z= $-CH_2-CH_2-$, as it is shown in the scheme, but may also be easily converted into derivatives in which Z= $-CHCl-CH_2-$ $-CHF-CH_2-$, $-CHCN-CH_2-$, $-CH(OCH_3)-CH_2-$ by treating with thionyl chloride followed by reaction with KF or KCN or $CH_3ONa$.

The amine of the formula 11, where Z is the $-C_6H_4-CHY-CH_2$ group, can be obtained by treating 1-phenyl-4-alkylbicyclo[2,2,2]octane with the acid chloride 36 and converting the resulting ketone according to the scheme:

35

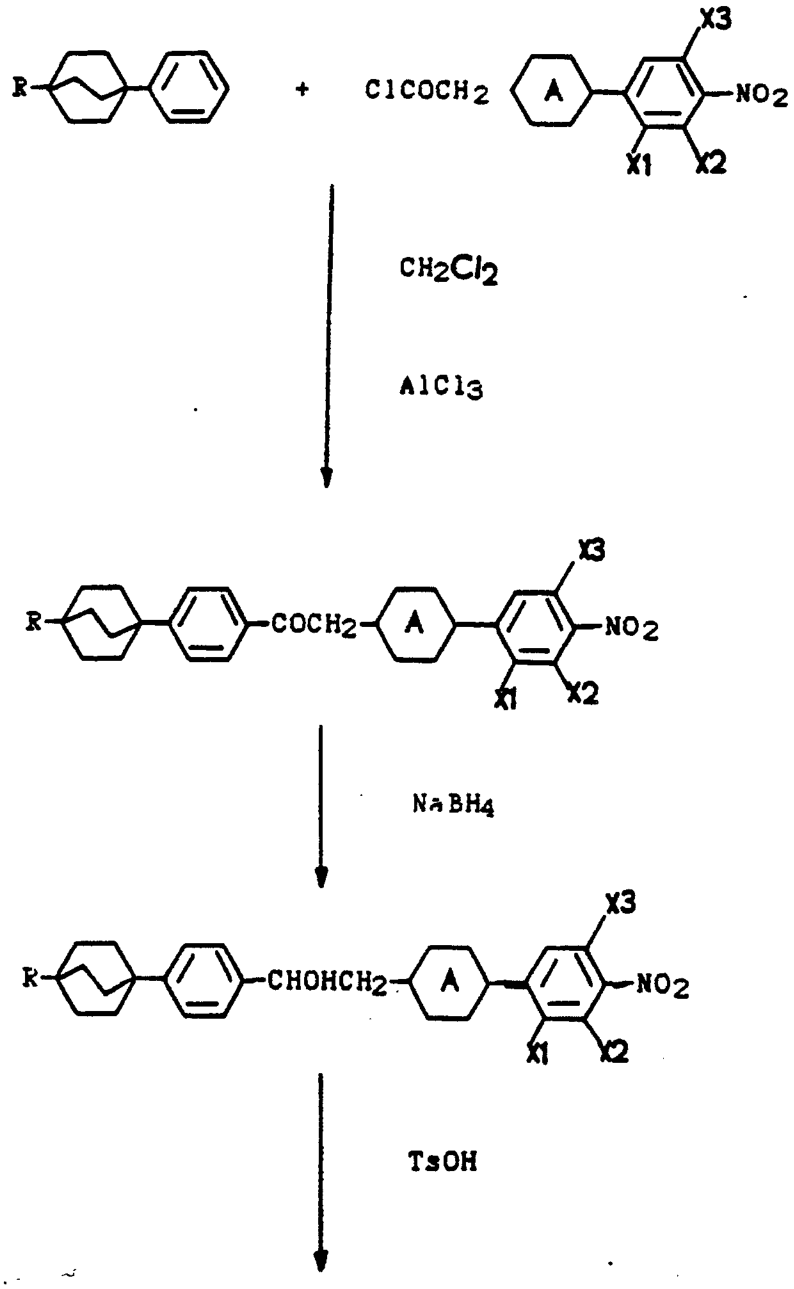


36

37

38

39

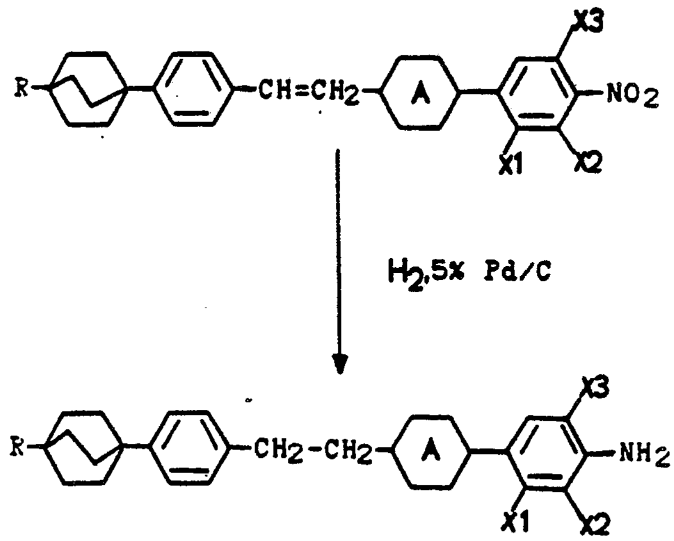


11

The isothiocyanates that are the object of the present invention reveal stable nematic phases like the analogous compounds with the cyano group, however, in distinction from the cyano compounds that sometimes have a wider range of the nematic phase thanks to the lower melting points observed for certain alkyl chains. Especially favourable properties in this respect are revealed by the compounds of formula 1 in which the alkyl chain has an even number of carbon atoms, for instance it is a hexyl or butyl group (n = 6 or n = 4). Compounds 1 are also characterized by a significantly lower viscosity, e.g. the viscosity of compounds 1a at 20 °C is of the order of 20-30 mPa.s, while the viscosity of the analogous cyano compounds is of the order of 100 mPa*s. Thank to the low melting enthalpies the solubility of the isothiocyanates in other classes of liquid crystalline compounds is very good. The phase transition temperatures of isothicyanates (1a and 1b) and of the correspondnig cyano compounds are compared in Table 1.

Tabl 1

| Nr | R | Z | m | -NCS | -CN |
|---|---|---|---|---|---|
| 1 | $C_3H_7$- | | O | Cr103(N88)I | Cr66.5N88I |
| 2 | $C_4H_9$- | | O | Cr93.5(N82.5)I | Cr75.5N85I |
| 3 | $C_5H_{11}$- | | O | Cr71N98I | Cr62N100I |
| 4 | $C_6H_{13}$- | | O | Cr50.5N89I | Cr72N86I |
| 5 | $C_8H_{17}$- | | O | Cr50.5N87I | Cr52N90I |
| 6 | $C_4H_9$- | -COO | O | Cr73N101I | Cr99(N96)I |
| 7 | $C_5H_{11}$- | -COO | O | Cr74.5N116.5I | Cr89N109I |
| 8 | $C_6H_{13}$- | -COO- | O | Cr50.5N106I | Cr77N102I |
| 9 | $C_8H_{17}$- | -COO | O | | |
| where: Cr, N and I is the crystalline, nematic and isotropic phase, respectively. | | | | | |

The liquid crystalline device in which the mixture of the present invention is used consists of two plates coated with a conducting layer spaced by a insulating material, one of which at least is transparent and has on its internal wall etched electrodes which allow application of an electric field to the layer of the liquid crystalline mixture, containing at least one compound of formula 1, placed between the plates.

The examples given below illustrate the methods of obtaining new compounds of the general formula 1, as well as of preparation of liquid crystalline mixtures containing these compounds. The examples are given to illustrate the invention rather than to limit its range. The temperatures are in all instances given in °C.

Example 1

Production of 4-(4′-n-hexylbicyclo[2,2,2]octyl-1)isothiocyanatobenzene

a) 70 g (0.306 mole) of 1-chloro-4-n-hexylbicyclo[2,2,2]-octane obtained according to Adomenas (Zh. Org. Khim, 19, 2366, (1983)). are added dropwise to a mixture of 500 $cm^3$ of benzene and 20 g (0.122 mole) anhydrous $FeCl_3$ with vigorous stirring. After adding is terminated the contents are heated to 60°, then cooled to the room temperature and pourred into water with ice. The organic layer is separated, washed with water and dried with anhydrous $MgSO_4$. After filtration excess of benzene is distilled off and the residue distilled under vacuum, 0.01 mmHg. The yield of 1-phenyl-4-hexylbicyclo[2,2,2]octane is 72.3 g (87.6 % of theoretical) with m.p. 38-39°.

b) The mixture of 16 g of anhydrous aluminium chloride, 100 $cm^3$ of methylene chloride, 8.6 g (0.11 mole) acetyl chloride are cooled to -5° upon which the solution obtained by disolving 27 g (0.1 mole) 1-phenyl-4-hexylbicyclo[2,2,2]octane in 100 $cm^3$ of methylene chloride is added dropwise and stirring is continued for 3 h, the temperatur by being kept at -5°. Next the contents are pourred into a mixture of water and ice, the organic layer is separated, washed with water, dried with anhydrous $MgSO_4$. After filtration the solvent is distilled off. The solid residue is recrystallized from hexane the yield 4-(4-n-hexylbicyclo[2,2,2]octyl-1)acetophenone of m.p. 82-83° being 22 g (70.5% theoretical).

c) 11 g (0,207 mole) of sodium acetate added portionwise with stirring to the mixture composed of 21.5 g (0.069 mole) of 4-(4-n-hexylbicyclo[2,2,2]octyl-1)acetophenone, 7.2 g (0.104 mole) of hydroxylamine hydrochloride and 300 $cm^3$ ethanol heated to the boil. The contents is next refluxed for 4 h, cooled to room temperature poured into water and the precipitated crystalline oxime is filtered off. After recrystallization from ethanol the yield of 4-(4-n-hexylbicyclo[2,2,2]octyl-1)acetophenone oxide of m.p. 175-176° of is 18 g ( 79.9 % theoretical).

d) The 18 g (0,057 mole) of the oxime obtained in (c) are added with stirring to the mixture composed of 17.8 g (0.086 mole) of phosphorus pentachloride and 200 $cm^3$ of methylene chloride at a rate ensuring that the temperature is kept in the range of 15-20°. Stirring is continued at room temperature for a further 4 h and next the contents are pourred on finely crushed ice. Methylene chloride is distilled off from the mixture and the crystalline 4-(4-n-hexylbicyclo[2,2,2]octyl-1)acetaniline of m.p. 195° is filtered off.

e) the crude acetanilide obtained in (d) is heated at 95° with 250 $cm^3$ of 20% hydrochloric acid for 4 h. The resulting 4-(4-n-hexylbicyclo[2,2,2]octyl-1)aniline hydrochloride is filtered off, washed with water and-refluxed for 2 h with 250 $cm^3$ of 20% sodium hydroxide. Next the amine is extracted with benzene and after drying over NaOH benzene is distilled off and the residue is recrystallized from ethanol. The yield of the obtained 4-(4 -n-hexylbicyclo [2,2,2]octyl-1)aniline of m.p. 87° is 12.5 g (76.8 % of theoretical).

f) A chloroform solution containing 12 g (0.042 mole) of 4-(4-n-hexylbicyclo[2,2,2]octyl-1)aniline is added dropwise with stirring to the mixture of 8.4 g (0.084 mole) of calcium carbonate, 50 $cm^3$ of water, 50 $cm^3$ of chloroform and 5.8 g (0.05 mole) of thiophosgene cooled to +50, the temperature being kept at +50. Stirring is further continued for 3 h, the contents are filtered and the organic layer is separated and dried over anhydrous $MgSO_4$. After distilling off chloroform the crude product is recrystallized twice from a methanol-ether mixture. The yield of 4-(4-n-hexylbicyclo[2,2,2]octyl-1)isothiocyanatobenzene is 11 g (80% of theoretical) and the phase transition points of this substance are: Cr50.5N89I.

The compounds:
4-(4-n-propylbicyclo[2,2,2]octyl-1)isothiocyanatobenzene, Cr103.5(N88)I
4-(4-n-butylbicyclo[2,2,2]octyl-1)isothiocyanatobenzene, Cr93(N82)I
4-(4-n-pentylbicyclo[2,2,2]octyl-1)isothiocyanatobenzene, Cr74N99.51
4-(4-n-octylbicyclo[2,2,2]octyl-1)isothiocyanatobenzene, Cr50N87I
4-(4-n-hexylbicyclo[2,2,2]octyl-1)-4′-isothiocyanatobiphenyl, 1-[4-(4-n-octylbicyclo[2,2,2]octyl-1]-2-[(4-isothiocyanatophenyl)cyclohexyl]ethane are obtained analogously.

Example 2

Synthesis of the 4-isothiocyanatophenyl ester of 4-n-hexyl bicyclo[2,2,2]octane-1-carboxylic acid

a) The mixture containing 5 g (0,021 mole) of 4-n-hexylbicyclo[2,2,2]octane-1-carboxylic acid, 5 $cm^3$ of oxalyl chloride, and one drop of DMF is left to stand at room temperature until the evolution of gases ceases. Then it is heated fo 2 h at 50°, and the excess of oxalyl chloride is distilled off. The residual crude acid chloride is dissolved in 20 $cm^3$ of benzene, and the solution is added dropwise to a mixture composed of 3.5 g of 4-nitrophenol, 5 $cm^3$ of pyridine, and 20 $cm^3$ of benzene. The mixture is left to stand for 24 h at

room temperature upon which it is poured onto water. The organic layer is separated, washed with 5 % hydrochloric acid and next with a 5 % solution of sodium bicarbonate and water, then dried with anhydrous magnesium sulfate. Benzene is distilled off and the crude ester is crystallized from ethanol. The 4-nitrophenyl ester of 4-n-hexylbicyclo[2,2,2]octane-1-carboxylic acid with phase transition point Cr55 N64.5 I is obtained with a yield of 5 g (66 % of theoretical).

The compounds:

4-nitrophenyl ester cf 4-butylbicyclo[2,2,2]octane-1-carboxylic acid Cr85I

4-nitrophenyl ester of 4-pentylbicyclo[2,2,2]octane-1-carboxylic acid

4-nitrophenyl ester of 4-octylbicyclo[2,2,2]octane-1-carbo xylic acid Cr60N731 and

3-fluor-4-nitrophenyl ester of 4-octylbicyclo[2,2,2]octane-1 carboxylic acid are obtained in the same way.

b) The obtained ester is suspended in 100 $cm^3$ of ethanol, 0.5g of 5 % palladium deposited on carbon is added, and hydrogenation is conducted under atmospheric pressure. When the reaction mixture ceases to absorb hydrogen it is heated to boil, the catalyst is filtered off, and the filtrate is left to crystallize. the 4-aminophenyl ester of 4-n-hexylbicyclo[,2,2,2]octane-1-carboxylic acid with phase transition points Cr104$S_A$113.5I is obtained with a yield of 4.1 g (90 % of theoretical).

The compounds:

4-aminophenyl ester of 4-butylbicyclo[2,2,2]octane-1-carboxylic acid, Cr138.5I

4-aminophenyl ester of

4-pentylbicyclo[2,2,2]octane-1-carboxylic acid, Cr123($S_A$108)I

4-aminophenyl ester of 4-octylbicyclo[2,2,2]octane-1-carboxylic acid, Cr84$S_A$119I

3-fluor-4-aminophenyl ester of 4-octylbicyclo[2,2,2]octane--1- carboxylic acid,

are obtained in a similar manner.

c) The mixture containing 5 g of calcium carbonate, 15 $cm^3$ of water, 30 $cm^3$ of chloroform is cooled to about 3° when 1.5 $cm^3$ (0.0196 mole) of thiophosgene is added. Next the chloroform solution of 4.1 g of the amino ester obtained in step (b) are added dropwise with vigorous stirring, the temperature being kept at 3°. Stirring is continued for 1 h at 3° after the adding is terminated and then for 3 h at room temperature. The excess of calcium carbonate is filtered off and the organic phase is separated and dried with anhydrous magnesium sulfate. After distilling off chloroform the ester is recrystallized from ethanol and hexane. The 4-isothiocyanatophenyl ester of 4-n-hexylbicyclo [2,2,2]octane-1-carboxylic acid with phase transition points Cr50.5N106I is obtained with a yield of 2.1 g (45% of theoretical).

The compounds: 4-isothiocyanatophenyl ester of 4-butylbicyclo[2,2,2]octane-1-carboxylic acid, Cr73N101I,

4-isothiocyanatophenyl ester of

4-pentylbicyclo[2,2,2]octane-1-carboxylic acid, Cr74.5N116.5I 4-isothiocyanatophenyl ester of

4-octylbicyclo[2,2,2]octane-1-carboxylic acid, Cr58N102I 4-isothiocyanatophenyl ester of 3-fluoro-4-octylbicyclo[2,2,2]octane-1-carboxylic acid, Cr68.5N92I

are obtained analogously.

## Example 3

(4-hexylbicyclo[2,2,2]octyl-1)methyl-4′-isothiocyanatophenyl ether

a) The mixture containing 4.17 g (0,03 mola) 4-nitrophenol, 11,34 g (0,03 mole) of 1-hydroxymethyl-4-hexylbicyclo[2,2,2]·octyl 4-toluenesulfonate, 12,4g (0.09 mole) of anhydrous potassium carbonate, and 60 $cm^3$ DMF was heated with mixing at 130° for 6 h. Next is poured into 300 $cm^3$ of water. The separated oil is extracted three times with 50 $cm^3$ portions of benzene, the benzene extracts are washed with a 5% solution of sodium hydroxide, water, dried with anhydrous $MgSO_4$, and filtered. Benzene is distilled off in a vacuum evaporator. The crude 4[α (4-hexylbicyclo[2,2,2]octy-1)]-nitroanisol is obtained with a yield of 5 g, and is subjected to reduction in (b) without purification.

b) 5 g (0.0144 mole) of 4[α (4-hexylbicyclo[2,2,2]octy-1)]-nitroanisol, 2.26 g (0.036 mole) of 80% hydrazine hydrate, and 100 $cm^3$ of ethanol are heated to 40° with stirring. On reaching this temperature a freshly prepared suspension of Raney nickel is added. After the reaction is terminated the nickel is filtered off and the filtrate is concentrated to complete evaporation. The residue is dissolved in 50 $cm^3$ of chloroform and 3 $cm^3$ of concentrated hydrochloric acid are added with stirring. The precipitating 4[α(4-hexylbicyclo[2,2,2]octyl-1)anisidine hydrochloride is filtered and treated with a 20 % solution of NaOH. The isolated amine is recrystallized from ethanol. The yield is 2.5 g (55.1 % of theoreetical) and the melting poit of the product is 75-77°.

c) 10 $cm^3$ of water, 10 $cm^3$ chloroform, 1,6 g (0.016 mole) of calcium carbonate and 1.05 g (0.091 mole) of thiophosgene were cooled with stirring to +3°. At this temperature the solution of 2.5 g (0.079 mole) of 4[α(4-heksylobicyklo[2,2,2]oktylo-1)anisidine in 30 $cm^3$ chloroform added dropwise. Next the unreacted calcium carbonate filtered off, and the chloroform layer separated, dried with anhydrous magnesium sulfate and filtered through silica gel. The product is recrystallized from a methanol:acetone mixture. The yield of (4-hexylbicyclo[2,2,2]octyl-1)methyl-4′-isothiocyanatophenylether is 1.5 g (53 % of theoretical, and the phase transition points of the product are Cr77° (N75.5°)I.

Example 4

4-(trans-4-n-hexylcyclohexyl)-1-isothiocyanatobenzene has its clearing at 43° and viscosity $\eta$20° = 13,3 mPa•s.
The equimolar mixture of 4-(trans-4-n-hexylcyclohexyl-1)-isothiocyanatobenzene and 4-(4-n-hexylbicyclo-[2,2,2]octyl-1) isothiocyanatobenzene has a clearing point at 65,5° and viscosity $\eta$20° = 17,3mPa•s

Example 5

The ternary mixture A of the composition in wt. %:
4-(trans-4-n-propylcyclohexyl)-1-isothiocyanatobenzene 40
4-(trans-4-n-hexylcyklohexyl)-1-isothiocyanatobenzene 42
4-(trans-4-n-octylcyclohexyl)-1-isothiocyanatobenzene 18
has the clearing point at 41,5° and viscosity $\eta$20° = 11 mPa•s.
The mixture of the following composition in wt. %:
mixture A 90
4-(4-n-propylbicyclo[2,2,2]octyl-1)isothiocyanatobenzene 10
has the clearing point at 49,5° (by 8° C higher than A) and viscosity $\eta$20° = 12,8 mPa•s (only by 1,8 mPa•s higher than that of A).

Example 6

The mixture of the following composition in wt. %:
mixture A 67,97
4-(4-n-propylbicyclo[2,2,2]octyl-1)isothiocyanatobenzene 6
4-(4-n-hexylbicyclo[2,2,2]octyl-1)isothiocyanato benzene 26
1-[4-(2-methylbutyl)biphenylyl-4′]-2-(4-isothiocyanatophenyl)ethane
optical active 0,03
is a nematic with clearing point at 54,5° and viscosity $\eta$20° = 15,6 mPa•s.

Example 7

The mixture of the following composition in wt. %:
mixture A 63
4-(4-n-hexylbicyclo[2,2,2]octyl-1)isothiocyanato benzene 25
4-isothiocyanatophenyl ester of 4-n-hexylbicyclo[2,2,2] octane-1-carboxylic acid 11
4-(2-methylbutoxy)phenyl 4-octyloxybenzoate optical active 1
is a nematic with clearing point at 56,5° C and viscosity $\eta$20° = 16,4 mPa•s.
threshold voltage 20° $V_{10\%}$ = 1.7 V
saturation voltage 20° $V_{90\%}$ = 2.5 V

Example 8

The mixture of the following composition in wt. %:

mixture A 64,97
4-(4-n-hexylbicyclo[2,2,2]octyl-1)isothiocyanatobenzene 20
1-[4-(trans-4′-n-butylcyclohexyl(phenyl]-2-[trans-4-(4′-isothiocyanatophenyl)cyclohexyl]ethane 15
1-[4-(2-methylbutyl)biphenyl-4′]-2-[4-isothiocyanato phenyl]ethane 0,03
is a nematic with clearing point at 87° C and viscosity $\eta$20° = 16,4 mPa•s, $V_{10\%}$ = 2.0 V, $V_{90\%}$ = 2.7 V

Example 9

The mixture of the following composition in wt. %:
4-(4-n-propylbicyclo[2,2,2]octyl-1)isothiocyanatobenzene 10
4-(4-n-hexylbicyclo[2,2,2]octyl-1)isothiocyanatobenzene 37
4-isothiocyanatophenyl ester of (4-n-hexylbicyclo[2,2,2]octane)-1-carboxylic acid 15
4-n-hexyloxyphenyl 4-n-butylbenzoate 37
4-(2-methylbutyloxy)phenyl 4-n-octyloxybenzoate
optical active 1
is a nematic from 46,5° to 71°, and below 46,5° has the smectic A phase.

Example 10

The mixture of the following composition in wt. %:
4-(trans-4-n-propylcyclohexyl-1)isothiocyanatobenzene 30.84
4-(trans-4-n-hexylcyclohexyl-1)isothiocyanatobenzene 30.72
4-(4-n-hexylbicyclo[2,2,2]octyl-1)isothiocyantobenzene 27.8
4-(4-n-pentylbicyclo[2,2,2]octyl-1)isothiocyanatobenzene 10.64
has the clearing point at 58,8°, viscosity $\eta$20° = 13,5 mPa•s, $\Delta\epsilon$ = 6,6 and $\Delta\eta$ = 0,19
threshold voltage 20° $V_{10\%}$ = 1.7 V
saturation voltage 20° $V_{90\%}$ = 2.5 V

Example 11

The mixture of the following composition in wt. %:
4-(trans-4-n-propylcyclohexyl-1)isothiocyanatobenzene 30,84
4-(trans-4-n-hexylcyclohexyl-1)isothiocyanatobenzene 30,72
4-(4-n-hexylbicyclo[2,2,2]octyl-1)isothiocyanatobenzene 27,77
4-(4-n-pentylbicyclo[2,2,2]octyl-1)isothiocyanatobenzene 10,64
1-[4-(2-methylbutyl)biphenylyl-4′]-2-(4-isothiocyanatophenyl)ethane 0,003
has the nematic phase from -30 to 58.5° and it has at temperature 20° bulk viscosity $\eta$20° = 13.5 mPa•s, $\Delta\epsilon$ = 5.8 $\Delta\eta$ = 0.2, threshold voltage $V_{10\%}$ = 1.7 V, saturation voltage 20° $V_{90\%}$ = 2.5 V

Example 12

The mixture of the following composition in wt.%:
4-(trans-4-n-propylcyclohexyl-1)isothiocyanatobenzene 30.47
4-(trans-4-n-hexylcyclohexyl-1)isothiocyanatobenzene 30.32
4-(4-n-hexylbicyclo[2,2,2]octyl-1)isothiocyanatobenzene 27.48
4-(4-n-pentylbicyclo[2,2,2]octyl-1)isothiocyanatobenzene 7.36
4-(4-n-hexylbicyclo[2,2,2]octyl-1)isothiocyanatobiphenylyl-1)ethane 4.34
1-[4-(2-methylbutyl)biphenylyl-4′]-2-(4-isothiocyanatophenyl)ethane 0.03
has the nematic phase from -30 to 68° and it has at temperature 20° bulk viscosity $\eta$ 20° = 14.5 mPa•s, $\Delta\epsilon$ = 7.0, $\Delta\eta$ = 0.21, threshold voltage $V_{10\%}$ = 1.8 V, saturation voltage $V_{90\%}$ = 2.6 V.

Example 13

The mixture of the following composition in wt. %:
4-(4-n-propylbicyclo[2,2,2]octyl-1)isothiocyanatobenzene 15
4-(4-n-hexylbicyclo[2,2,2]octyl-1)isothiocyanatobenzene 45
4-(4-n-octylbicyclo[2,2,2]octyl-1)isothiocyanatobenzene 40
has the clearing point at 86° and bulk viscosity at temperature 20° $\eta 20° = 33$ mPa•s.

## Claims

1. Liquid crystalline compounds containing the bicyclo[2,2,2]octane ring and the phenylisothiocyanato group in their molecules of the general formula 1:

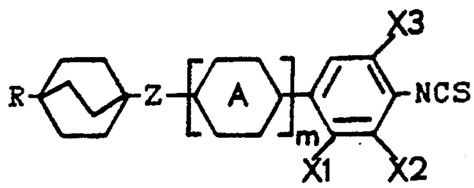

1

wherein: R is a normal or branched alkyl $H_{2n+1}C_n$- or alkenyl $H_{2n-1}C_n$- group containing up to 15 carbon atoms, some of the hydrogen atoms in the alkyl chain being sometimes substituted by a fluorine atom, Z is a single bond or -COO-, -OOC-, $-CH_2O-$ or -C≡C- group, the ring A is a 1,4-disubstituted benzene ring or 2,5-disubstituted pyrimidine ring or a 2,5-disubstituted pyridine ring or a 2,5-disubstituted 1,3-dioxane ring, m stands an integer 0 or 1, X1, X2 and X3 are identical or different and each of them denotes a hydrogen atom or a fluorine atom or a chlorine atom or a bromine atom or a cyano group, however, if m = 1 and the ring A is not a benzene ring then Z is also a $-CH_2CO-$, $-CO-CH_2-$, -CH = CH-, $-CHYCH_2-$, $-CH_2-CHY-$, $-C_6H_4-CH_2-CHY-$, $-C_6H_4-CHY-CH_2-$ or $-C_6H_4-C≡C-$ group where Y is a hydrogen atom or a fluorine atom or a chlorine atom or a cyano group or a methoxy group or a hydroxy group.

2. A compound according to claim 1, wherein Z is a single bond, m assumes the value 0, and X1, X2, X3 are hydrogen atoms, of the general formula 1a:

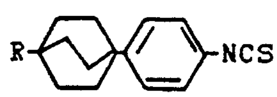

1a

wherein R is an alkyl chain with 1 to 10 carbon atoms.

3. A compound according to claim 1, wherein Z is the -COO-group, m assumes the value of 0, and X1, X2, X3 are hydrogen atoms, of the general formula 1b:

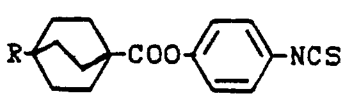

1b

wherein R is a normal alkyl chain with 1 to 10 carbon atoms.

4. A compound according to claim 1, wherein Z is the $-CH_2O-$ group, m assumes the value 0, and X1, X2, X3 are hydrogen atoms, of the general formula 1c:

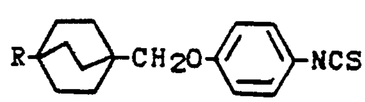

1c

wherein R is a normal alkyl chain with 1 to 10 atoms.

5. A compound according to claim 1, wherein Z is the -C≡C-group, m assumes the value 0, and X1, X2, X3 are hydrogen atoms, of the general formula 1d:

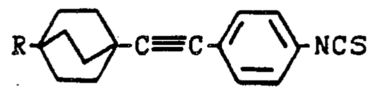

1d

wherein R is a normal alkyl chain with 1 to 10 carbon atoms.

6. A compound according to claim 1, wherein Z is the -COO-group, m assumes the value 0, and X1 and X2 are hydrogen atoms, and X3 is a fluorine atom, of the general formula 1e:

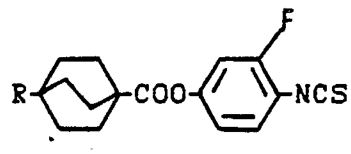

1e

where R is a normal or branched alkyl chain with 1 to 10 carbon atoms.

7. A compound according to claim 1, wherein Z is a single bond the ring A is a benzene ring, and m assumes the value 1, of the general formula 1f:

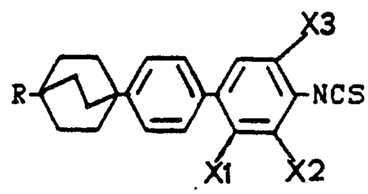

1f

wherein: X1, X2, X3 are identical or different and denote a hydrogen atom or a fluorine atoms, and R is a normal alkyl chain with 1 to 10 carbon atoms.

8. A compound according to claim 1, wherein Z is a single bond the ring A is a pyrimidine ring, and m assumes the value 1, of the general formula 1g:

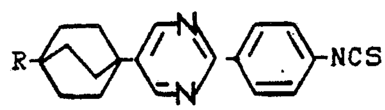

1g

wherein: X1, X2, X3 are hydrogen atoms, and R is a normal alkyl chain with 1 to 10 carbon atoms.

9. A compounds according to claim 1, wherein Z is the $-CH_2CH_2-$ group, the ring A is a cyclohexane ring, and m assumes the value 1, of the general formula 1h:

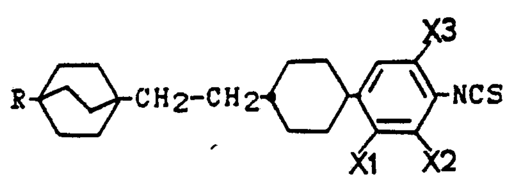

1h

wherein X1, X2, X3 are identical or different and denote a hydrogen atom, a fluorine atom, or a cyano group, and R is a normal alkyl chain with 1 to 10 carbon atoms.

10. A compounds according to claim 1, wherein Z is the -COO-group, the ring A is a cyclohexane ring, and m assumes the value of 1, of the general formula 1i:

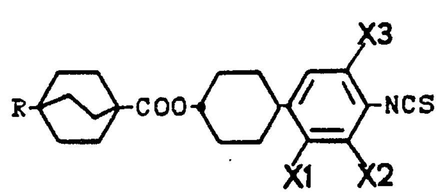

1i

wherein X1, X2, X3 are identical or different and each is a hydrogen atom or a fluorine atom or a cyano group, and R is a normal alkyl chain with 1 to 10 carbon atoms.

11. A compounds according to claim 1, wherein Z is the -OOC-group, the ring A is a cyclohexane ring, and m assumes the value 1, of the general formula 1j:

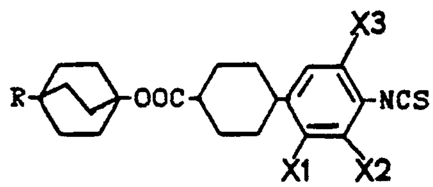

1 j

wherein X1, X2, X3 are identical or different and each is a hydrogen atom or fluorine atom or·cyano group, and R is a normal alkyl chain with 1 to 10 carbon atoms.

12. A compound according to claim 1, wherein Z is the $-CH_2CH_2-$ group, the ring A is a pyrimidine ring, and m assumes the value 1, of the general formula 1k:

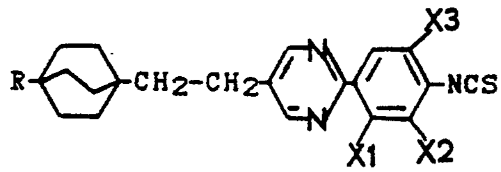

1k

wherein X1, X2, X3 are identical or different and each is a hydrogen atom or fluorine atom, and R is a normal alkyl chain with 1 to 10 carbon atoms.

13. A compounds according to claim 1, wherein Z is the phenyl-ethylene group ($-C_6H_4-CH_2-CH_2-$), the ring A is a cyclohexane ring, m assumes the value 1, of the general formula 11:

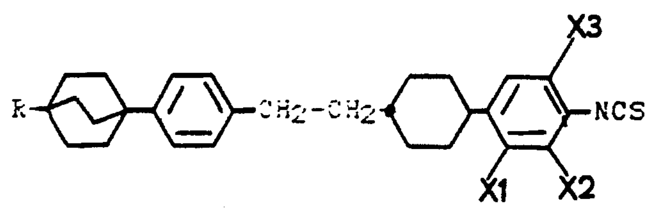

1 1

wherein X1, X2, X3 are identical or different and each is a hydrogen atom or fluorine atom or cyano group, and R is a normal alkyl chain with 1 to 10 carbon atoms.

14. A liquid crystalline mixture including at least two components, one of which is at least a compound according to claim 1 of the general formula 1.

15. A liquid crystalline mixture according to claim 14 characterized by that it includes dyes, solvents, and optically active compounds.

16. A liquid crystalline mixture according to claim 14 or 15 that includes compounds of the general formula 1 in proportions from 5 wt. % to 40 wt. %.

17. A liquid crystalline mixture according to claim 14 or claim 15 in which the second component is one compound or several compounds of the 4-(trans-4-n-alkylcyclohexyl)-1-isothiocyanatobenzene homologous series.

18. A liquid crystalline mixture according to claim 14 or claim 15 in which the second component is at least the one compound of the 1-[4-(trans-4-n-alkylcyclohexyl)phenyl]-2-[4-(trans-4-isothiocyanatophenyl)-cyclohexyl]ethane homologous series, and at least one compound of the 4-[trans-4-n-alkylcyclohexyl)-1-isothiocyanatobenzene homologous series.

19. The method of manufacturing the compounds according to claim 1 characterized by that the amine of formula 11, wherein R, Z, the ring A, and m are the same as in formula 1, is treated in an organic solvent, preferably benzene, with carbon disulfide in the presence of a tertiary amine, and the obtained in

the reaction crystalline trialkylammonium dithiocarbamate is isolated from the reaction mixture and subsequently treated with an acid chlorine, favourably ethyl chloroformate, in the presence of a tertiary amine, and the resulting product is isolated and purified by conventional methods.

20. The method of manufacturing of the compounds according to claim 1 characterized by that the amine of formula 11, wherein R, Z, the ring A and m have the same meaning as in formula 1, is treated with thiophosgene in the presence of a] hydrochlorine acceptor, preferably calcium carbonate, and the obtained isothiocyanate is isolated by conventional methods.

21. The improvement in an electrooptical cell comprising a liquid crystal dielectric disposed between two electrode plates, at least one of them is transparent, specific in that the applied liquid crystal dielectric is an admixture comprising compound of formula 1.

Fig. 1

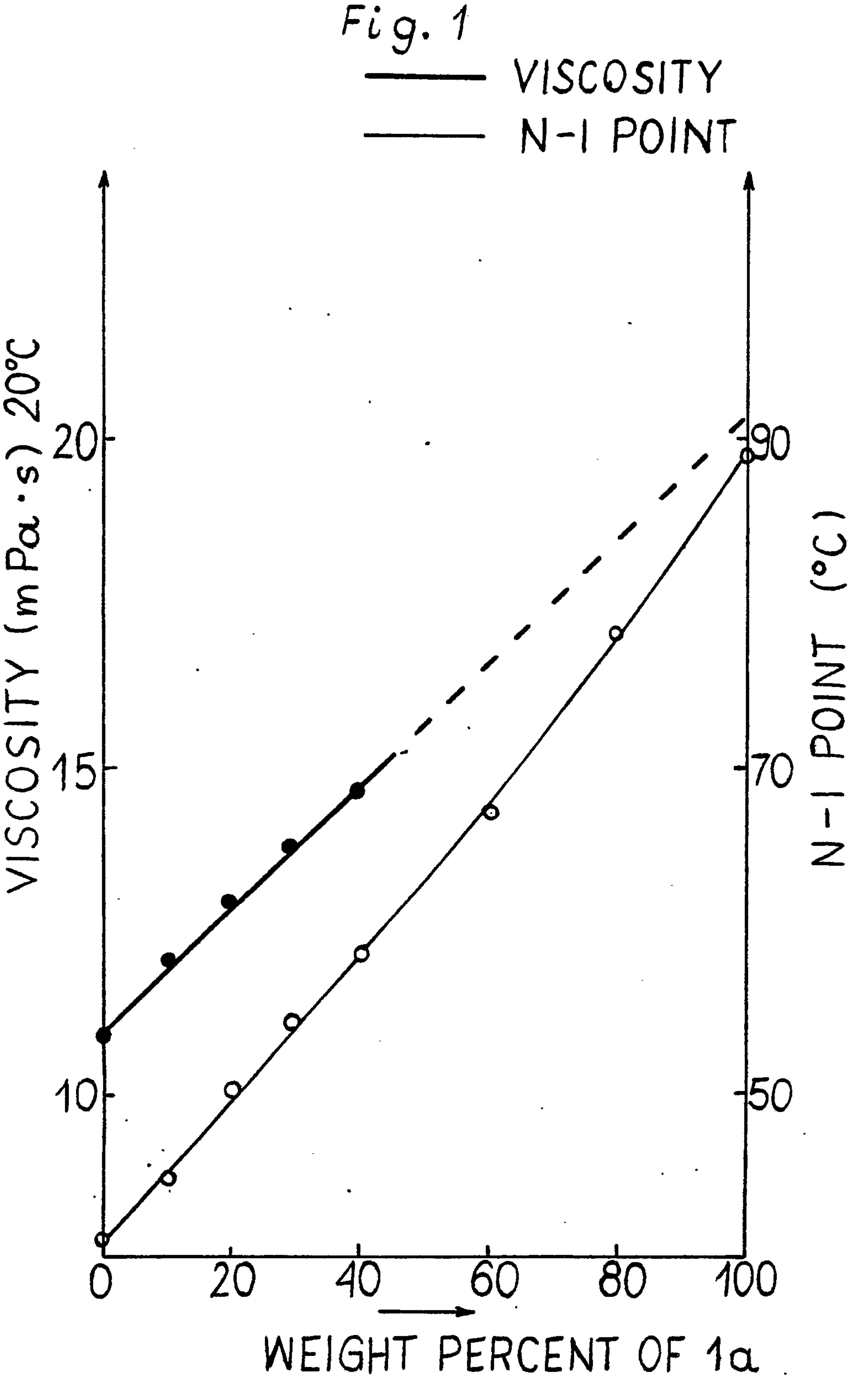
VISCOSITY
N-I POINT
VISCOSITY (mPa·s) 20°C
20
15
10
N-I POINT (°C)
90
70
50
0
20
40
60
80
100
WEIGHT PERCENT OF 1a